# EUROPEAN PATENT SPECIFICATION

(11) **EP 2 381 253 B1**
(45) Date of publication and mention of the grant of the patent: **25.12.2019**
(21) Application number: 11163029.9
(22) Date of filing: 19.04.2011
(51) Int. Cl.: G01N 33/18, C12Q 1/04, C12Q 1/22, C12Q 1/02

(54) **Apparatus for detecting toxicity in water using sulfur-oxidizing bacteria**
Vorrichtung zur Bestimmung von Toxizität in Wasser mithilfe von schwefeloxidierenden Bakterien
Appareil pour détecter la toxicité de l'eau au moyen de bactéries oxydant le soufre

(30) Priority: 22.04.2010 KR 20100037492; 16.02.2011 KR 20110013796
(43) Date of publication of application: 26.10.2011
(73) Proprietor: University-industry Cooperation Foundation Kangwon National University, Gangwon-Do 200-701 (KR)
(72) Inventor: Oh, Sang Eun, Chuncheon-si, Gangwon-do (KR); Hassan, Sedky, Chuncheon-si, Gangwon-do 200-701 (KR)
(74) Representative: ETL Wablat & Kollegen Patent- und Rechtsanwaltsgesellschaft mbH

(56) References cited:
- EP-A2- 0 242 225
- WO-A1-96/21723
- GB-A- 1 437 458
- KR-A- 20090 090 188
- US-A- 6 133 021
- US-A1- 2009 076 733
- SANG-EUN OH ET AL: "A novel biosensor for detecting toxicity in water using sulfur-oxidizing bacteria", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, vol. 154, no. 1, 8 February 2010 (2010-02-08), pages 17-21, XP028383919, ISSN: 0925-4005, DOI: 10.1016/J.SNB.2010.01.052 [retrieved on 2010-02-08]

## Description

### BACKGROUND OF THE INVENTION

### Field of the Invention

The present invention relates to an apparatus for detecting toxicity in water using sulfur-oxidizing bacteria, and more particularly to an apparatus for detecting toxicity in water using sulfur-oxidizing bacteria, which can provide accurate results of toxicity detection.

### Description of the Prior Art

Various methods for detecting toxic substances in water are known.

Of these, Korean Patent Laid-Open Publication No. 2009-28138 filed in the name of this applicant discloses an apparatus for detecting toxicity in water using sulfur particles.

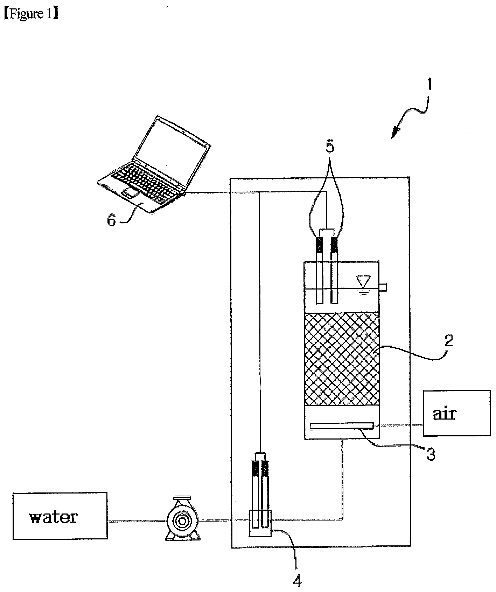

FIG. 1 is a schematic view showing the configuration of the water toxicity-detecting apparatus disclosed in the above publication. As shown therein, a water toxicity-detecting apparatus 1 comprises: a microbial reactor into which a water sample is introduced and which contains sulfur-oxidizing bacteria and sulfur particles; a first measurement unit 4 placed on the inlet side of the microbial reactor 2; and a second measurement unit 5 placed inside the microbial reactor 2. In the water toxicity-detecting apparatus 1, whether the water sample has toxicity is determined by comparing the electrical conductivity (EC) and pH values, measured in the first measurement unit 4, with those measured in the second measurement unit 5.

Namely, the invention disclosed in the above publication relates to a method and apparatus of detecting toxicity in water by determining the activity of sulfur-oxidizing bacteria on the basis of electrical conductivity and pH.

In this regard, the electrical conductivity of water allows prediction of the amount of the total salts and total ions dissolved in the water. However, in the water toxicity-detecting apparatus disclosed in the above publication, only the change in sulfate ions resulted from the oxidation of sulfur particles by sulfur-oxidizing bacteria occurs, and thus the change in electrical conductivity (EC) can be considered as the change in concentration of sulfate ions.

In principle, electrical conductivity is measured by placing two metals (electrodes) at a distance from each other in ion-containing water, applying voltage across the electrodes and measuring the generated current.

However, in the water toxicity-detecting apparatus 1 disclosed in the above publication, because the electrical conductivity-measuring unit and pH-measuring unit of the second measurement unit 5 are placed inside the microbial reactor 2, these measuring units can come into contact with air bubbles generated by aeration of the water sample and air, which are supplied through the bottom of the microbial reactor 2, thus causing fluctuations in the measured values of pH and EC.

In particular, the electrical conductivity-measuring unit has a problem in that, when air bubbles enter between the two electrodes, the data will fluctuate instantaneously, thus making it difficult to obtain accurate experimental values.

Also, the sulfur-oxidizing bacteria present in the reactor are autotrophic microorganisms that use carbon dioxide as a carbon source and require micronutrients such as nitrogen, phosphorus and potassium. For this reason, the water toxicity-detecting apparatus disclosed in the above publication is not effective in detecting toxicity in highly purified water containing little or no carbon dioxide and nutrients.

Moreover, in the case where a water sample has a very high pH and low alkalinity, the activity of sulfur-oxidizing bacteria that prefer low pH decreases, thus making it difficult to obtain accurate experimental data. Thus, in this case, a means capable of adjusting the pH of the water sample is required.

In addition, in the case where the sulfur-oxidizing bacteria present in the reactor are inactivated by toxic substances, it is impossible to continuously detect toxicity in an additional water sample. Namely, the sulfur particles should be replaced, and a time of about 2-14 days is required to reactivate the sulfur-oxidizing bacteria. During reactivation of the sulfur-oxidizing bacteria are reactivated, it is impossible to detect toxicity in the water sample.

### [Prior art documents]

### [Patent documents]

Patent document 1: Korean Patent Laid-Open Publication No. 2009-28138
Patent document 2: Korean Patent Laid-Open Publication No. 2009-108306

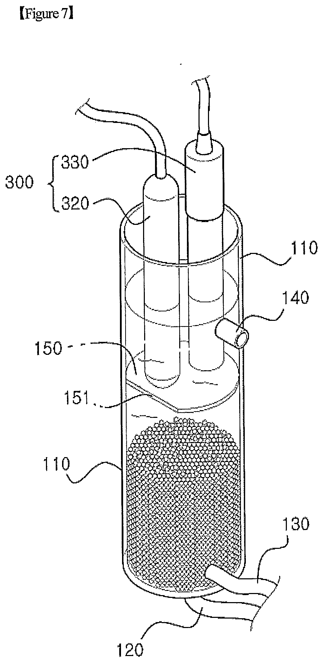

It is already known a novel biosensor for detecting toxicity in water using sulfur-oxidizing bacteria containing a reactor. The reactor is a tube in whose upper part probes for measuring pH and EC are arranged. In the water air is introduced by an air pump. The air bubbles rise adjacent to the probes, thereby not disturbing the measurement (Sang-Eun Oh et al.: "A novel biosensor for detecting toxicity in water using sulfur-oxidizing bacteria", International Journal Devoted to Research and Development of Physical and Chemical Transducers, vol. 154, pages 17-21). The measured values of pH and EC are recorded in a computer. The apparatus is depicted in Figure 7 of the present disclosure.

### SUMMARY OF THE INVENTION

Accordingly, the present invention has been made in view of the problems occurring in the prior art, and it is an object of the present invention to provide an apparatus for detecting toxicity in the water using sulfur-oxidizing bacteria, in which a measurement unit is not affected by air bubbles generated by air aeration in a microbial reactor, so accurate experimental results can be obtained.

Another object of the present invention is to provide an apparatus for detecting toxicity in water using sulfur-oxidizing bacteria, which can provide accurate experimental results even when extremely clean water, extremely contaminated water or a water sample having a very high pH is tested.

Still another object of the present invention is to provide an apparatus for detecting toxicity in water using sulfur-oxidizing bacteria, which further comprises an additional reactor that allows toxicity detection to be continuously performed even when sulfur-oxidizing bacteria present in one reactor are inactivated.

To achieve the above objects, the present invention provides an apparatus for detecting toxicity in water using sulfur-oxidizing bacteria, the apparatus comprising: a microbial reactor comprising a reactor body which contains sulfur-oxidizing bacteria and in which sulfur particles are oxidized by the sulfur-oxidizing bacteria in the presence of oxygen to form sulfate ions, an inlet through which a water sample is introduced into the reactor body, and an air inlet through which air is supplied to the reactor body; a measurement unit for measuring the pH and electrical conductivity (EC) of the water sample containing the sulfate ions produced in the reactor body, in which the measurement unit is placed in a separated space so as not to be affected by air bubbles generated due to supply of air to the reactor body, the separated space communicating with the reactor body so that the water sample containing the sulfate ions produced in the reactor body is introduced into the measurement unit; and a control unit which compares the electrical conductivity (EC) and pH values measured in the measurement unit with reference values to determine whether the water sample has toxicity.

In the apparatus of the present invention, the separated space in which the measurement unit for measuring pH and electrical conductivity (EC) are placed is defined by a measurement chamber formed integrally with the outer surface of the reactor body, and a through-hole through which the water sample passes may be formed in the reactor body provided with the measurement chamber.

Meanwhile, the apparatus of the present invention may further comprise a pH-adjusting unit which is placed on one side of the inlet of the microbial reactor and serves to supply an acidic substance to the water sample being supplied to the microbial reactor so as to adjust the pH of the water sample within the reference range.

Furthermore, the apparatus of the present invention may further comprise an auxiliary microbial reactor which is placed on one side of the microbial reactor and contains active sulfur-oxidizing bacteria, in which the auxiliary microbial reactor serves to detect toxicity in the water sample supplied from the microbial reactor when the sulfur-oxidizing bacteria in the microbial reactor were inactivated due to toxic substances.

Also, the auxiliary microbial reactor is operated by supplying minimum amounts of water and air thereto while the microbial reactor is used to detect toxicity in the water sample, and whether the sulfur-oxidizing bacteria have activity is monitored by measuring electrical conductivity.

In addition, the apparatus of the present invention may further comprise an auxiliary microbial reactor which is placed on one side of the microbial reactor, in which the auxiliary microbial reactor and the microbial reactor are alternately supplied with the water sample and a diluted water sample to determine whether the water sample has toxicity.

### BRIEF DESCRIPTION OF THE DRAWINGS

The above and other objects, features and advantages of the present invention will be more clearly understood from the following detailed description taken in conjunction with the accompanying drawing, in which:
FIG. 1 is a schematic view showing the configuration of an apparatus for detecting toxicity in water using sulfur-oxidizing bacteria according to the prior art;
FIG. 2 is a schematic view showing the configuration of an apparatus for detecting toxicity in water using sulfur-oxidizing bacteria ;
FIGS. 3 and 4 are, respectively, combined and exploded perspective views of the microbial reactor and measurement unit of an apparatus for detecting toxicity in water using sulfur-oxidizing bacteria according to the present invention;
FIG. 5 is a perspective view showing the microbial reactor and measurement unit of an apparatus for detecting toxicity in water using sulfur-oxidizing bacteria not according to the present invention;
FIG. 6 is a horizontal cross-sectional view of the measurement chamber shown in FIG. 5;
FIG. 7 is a perspective view showing the microbial reactor and measurement unit of an apparatus for detecting toxicity in water using sulfur-oxidizing bacteria not according to the present invention;
FIGS. 8 to 13 are graphs showing the experimental results of detecting toxicity in actual stream water using the inventive apparatus for detecting toxicity using sulfur-oxidizing bacteria; and
FIG. 14 is a table showing the composition of minerals used in preparation of synthetic stream water.

### DETAILED DESCRIPTION OF THE INVENTION

Hereinafter, the present invention will be described in detail with reference to the accompanying drawings.

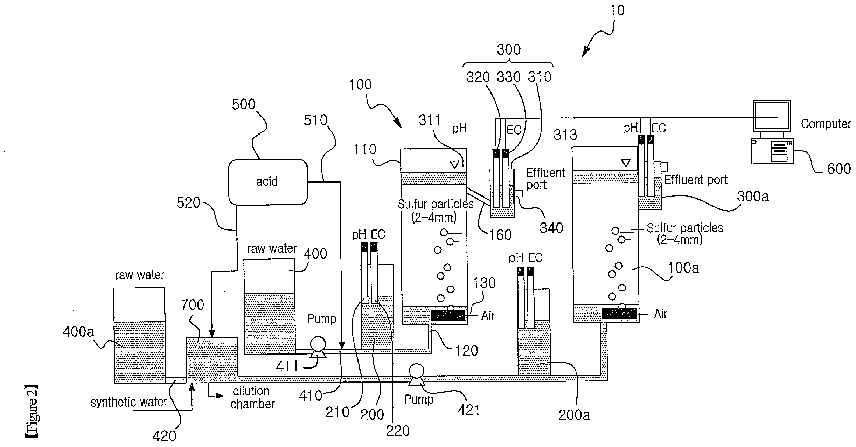

FIG. 2 is a schematic view showing the entire configuration of an apparatus for detecting toxicity in water using sulfur-oxidizing bacteria.

As shown therein, an apparatus 10 for detecting toxicity in water using sulfur-oxidizing bacteria according to the present invention comprises: a microbial reactor 100 which contains sulfur-oxidizing bacteria and in which the sulfur-oxidizing bacteria uses oxygen and sulfur particles as an electron acceptor and an electron donor, respectively, to form sulfate ions; a measurement unit 300 for measuring the pH and electrical conductivity (EC) of a water sample containing the sulfate ions generated in the microbial reactor 100; a raw water supply unit 400 for supplying the water sample; a pH-adjusting unit 500 which is disposed between the raw water supply unit 400 and an inlet 120 of the microbial reactor 100 and serves to adjust the pH of the water sample; and a control unit 600 for determining whether the water sample has toxicity on the basis of results measured in the measurement unit 300.

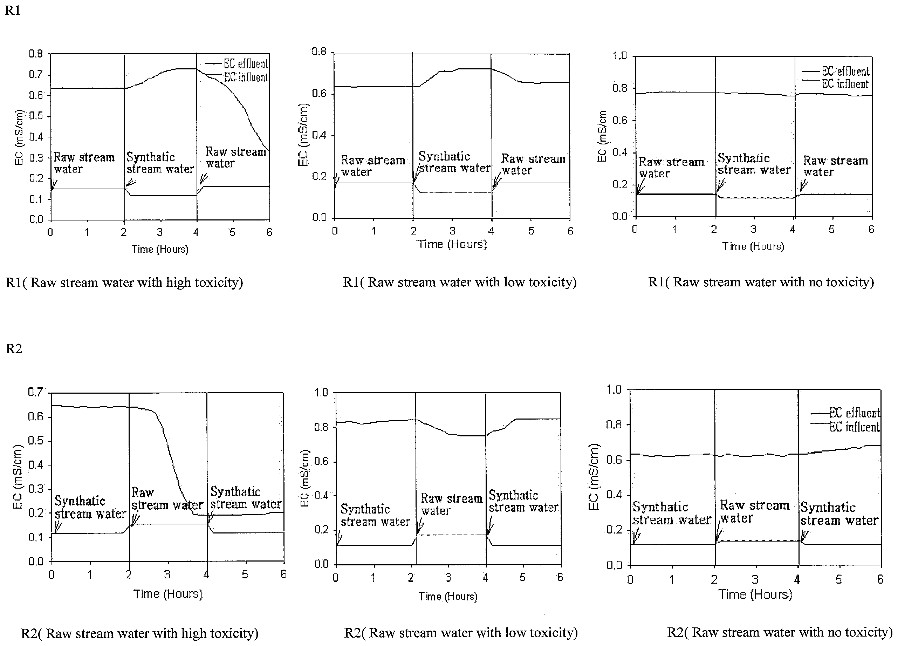

In the control unit 600, determining whether the water sample has toxicity on the basis of the results measured in the measurement unit 300 can be performed using various methods. For example, whether the water sample has toxicity can be determined by comparing the pH and electrical conductivity (EC) values of the water sample, which is introduced into the microbial reactor 100, with those measured in the measurement unit 300. Also, as shown in FIG. 13, whether the water sample has toxicity can be determined by comparing the change in EC of a synthetic stream water having no toxicity for a specific time (EC₁₂ₕ for control) with the change in EC of a water sample for a specific time (EC₁₂ₕ for sample), measured in the measurement unit 300.

The microbial reactor 100 contains sulfur particles and active sulfur-oxidizing bacteria, and thus sulfate ions are produced therein. The microbial reactor 100 comprises: a reactor body 110 in which a water sample and sulfur-oxidizing bacteria are placed; an inlet 120 which is connected to the lower region of the reactor body 110 and serves to introduce the water sample into the reactor body 110; an outlet 140 which is connected to the upper region of the reactor body 110 and serves to discharge the sample from the reactor body 110; and an air inlet 130 for supplying air to the interior of the reactor body 110.

In this regard, the water sample may be various kinds of water, including stream water, wastewater, and purified water.

The sulfur-oxidizing bacteria present in the reactor body 110 grow attached to the surface of the sulfur particles, and the sulfur particles are oxidized at a constant rate to produce sulfate ions. If the water sample has no toxicity, the concentration of the sulfate ions will increase due to the oxidation of the sulfur particles caused by the activity of the sulfur-oxidizing bacteria. On the other hand, if the water sample has strong toxicity, the activity of the sulfur-oxidizing bacteria will decrease so that they cannot oxidize the sulfur particles, and thus the concentration of the sulfate ions will not substantially change with time goes by. The microbial reactor 100 functions as a biosensor for detecting the presence of toxicity in the water sample on the basis of this principle.

The measurement unit 300 comprises a pH-measuring unit 320 and EC-measuring unit 330 for measuring the pH and electrical conductivity (EC) of the water sample containing the sulfate ions created by the oxidation of sulfur particles due to the activity of sulfur-oxidizing bacteria in the reactor body 110, and also comprises a space in which the pH-measuring unit 320 and the EC-measuring unit 330 are placed. The pH-measuring unit 320 and the EC-measuring unit 330 are provided in the form of electrodes.

In the present invention, the pH-measuring unit 320 and the EC-measuring unit 330 are placed in a space separated from the reactor body 110 so that they are not affected by air bubbles generated by supply of the water sample and air.

In this regard, the separated space and the reactor body 110 communicate with each other so that the water sample containing the sulfate ions produced in the reactor body 110 can be introduced into the separated space.

In the present invention, with respect to the structure of communication between the separated space and the reactor body 110, the separated space is formed outside the reactor body 110 in such a manner that it is integral with the reactor body 110.

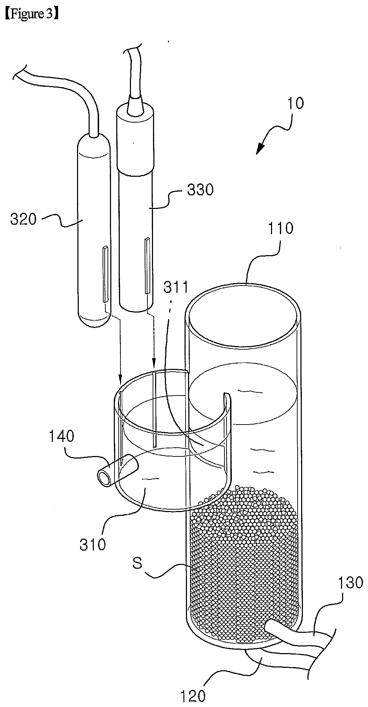

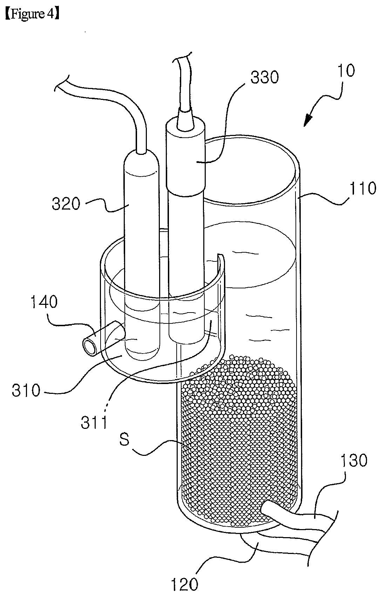

As shown in FIGS. 3 and 4, in the measurement unit 300 a measurement chamber 310 defining the separated space is provided outside the reactor body 110 in such a manner that it is integral with the reactor body 110, so that it is not affected by air bubbles generated by aeration by a water sample and air.

The measurement chamber 310 is provided integrally with the outside of the upper region of the reactor body 110, and a through-hole 311 through which a water sample can pass is formed in the reactor body 110 provided with the measurement chamber 310, so that the water sample flows from the reactor body 110 into the measurement chamber 310.

In the measurement chamber 310 into which the water sample is introduced through the through-hole 311, the pH-measuring unit 320 and the EC-measuring unit 330 are received so that they measure the physical properties of the water sample.

As described above, the pH-measuring unit 320 and the EC-measuring unit 330 is out of the path of movement of air bubbles generated by aeration of the water sample and air, which are introduced through the inlet 120 and the air inlet 130, respectively. Thus, air bubbles generated when the water sample and air flow can be prevented from coming into contact with and impacting the pH-measuring unit 320 and the EC-measuring unit 330. Accordingly, the pH-measuring unit 320 and the EC-measuring unit 330 can provide more accurate measurement data.

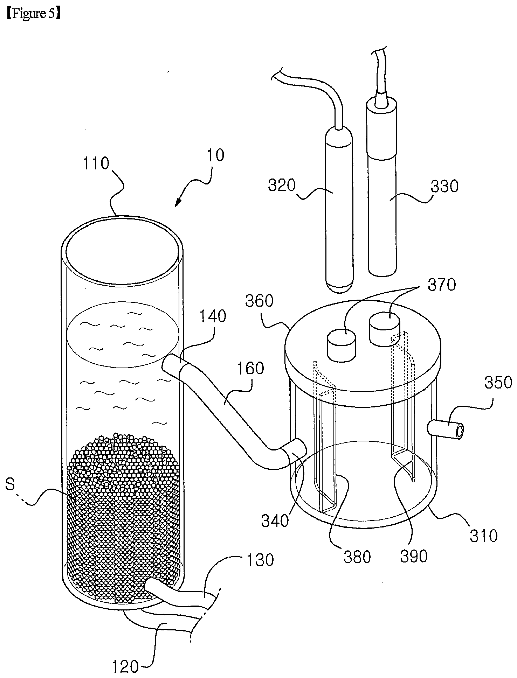

As shown in FIG. 5, in the measurement unit 300 not according to the present invention, the measurement chamber 310, which defines the separated space, and the reactor body 310 are spaced apart from each other and communicated with each other by a connection pipe 160.

Although the measurement chamber 310 is spaced apart from the reactor body 110, it is connected with the reactor body 110 by the connection pipe 160, and thus can be supplied with the water sample containing the sulfate ions produced in the reactor body 110.

The connection pipe 160 is connected with each of the outlet 140 of the microbial reactor 100 and the inlet 340 of the measurement chamber 310. In this regard, the inlet 340 of the measurement chamber 310 is placed at a level lower than the outlet 140 of the reactor body 110 so that the water sample containing the sulfate ions produced in the reactor body 110 can naturally flow into the measurement chamber 310.

Also, the top of the measurement chamber 310 is provided with a cover 360 capable of covering the measurement chamber 310. In the cover 360, two through-pipes 370 are provided so that the pH-measuring unit 320 and the EC-measuring unit 330 can be inserted into the measurement chamber 310 through the through-pipes 370. In addition, in the measurement chamber 310, an outlet 350 through which the water sample introduced into the measurement chamber 310 can be discharged is provided on the side opposite to the inlet 340.

The pH-measuring unit 320 and EC-measuring unit 330 for measuring the pH and electrical conductivity of the water sample containing the sulfate ions produced in the microbial reactor 100 are provided in such a manner that they are completely separated from the microbial reactor 100. Thus, air bubbles generated by air aeration in the microbial reactor 100 can be prevented from coming into contact with and impacting the pH-measuring unit 320 and the EC-measuring unit 330. Accordingly, the pH-measuring unit 320 and the EC-measuring unit 330 can provide more accurate measurement results.

The supply of the water sample from the microbial reactor 100 to the measurement chamber 310 can be naturally achieved without an additional power device such as a pump. Thus, the entire structure of the apparatus can be simplified so that the apparatus can be installed in a relatively narrow space and the installation cost of the apparatus can be reduced.

Meanwhile, the measurement chamber 310 does not contain sulfur-oxidizing bacteria and sulfur particles and does not require air aeration, unlike the microbial reactor 100, and the capacity thereof needs to be minimized compared to the microbial reactor 100. If the capacity of the measurement chamber 310 is excessively small, short circuiting can occur so that the water sample introduced into the measurement chamber will now have a sufficient retention time in the measurement chamber, and thus the electrical conductivity (EC) value of the water sample cannot appear. Also, because the water sample falls down from the microbial reactor 100 into the measurement chamber 310 due to the self-weight thereof, a large volume of flow can occur in the inlet of the measurement chamber 310, thus causing a vertical flow. This can adversely affect the pH-measuring unit 320 and the EC-measuring unit 330, thus making it difficult to obtain accurate measurement results.

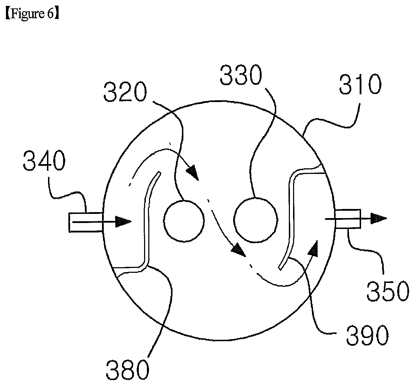

For this reason, in the apparatus not according to the present invention, as shown in FIGS. 5 and 6, a first baffle 380 made of a plate is placed vertically inside the inlet 340 of the measurement chamber 310 in such a manner as to extend from the bottom of the measurement chamber 310, so that it can reduce the flow rate of the water sample being into the measurement chamber 310 and suppress the occurrence of a large volume of flow.

In this regard, the first baffle 380 is placed at a certain distance from the inner wall of the measurement chamber 310. Specifically, one side of the first baffle 380 is placed in close contact with the inner wall of the measurement chamber 310, and the other side is open along the lengthwise direction of the first baffle 380 so that the water sample can flow along the first baffle and turn its flow direction, that is, a "U-turn" flow can be induced.

The first vertical baffle 380, which can suppress the occurrence of a flow rate of flow and reduce the flow rate of the water sample, is placed inside the inlet 340 of the measurement chamber 310. Accordingly, a suitable retention time of the water sample in the measurement chamber 310 can be ensured so that no short circuiting can occur while the occurrence of a vertical flow can be prevented, and thus more accurate measurement values can be obtained.

In addition, a second baffle 390 corresponding to the first baffle 380 is also placed inside the outlet 350 of the measurement chamber 310 so that the water sample can flow along the second baffle and turn its flow direction toward the outlet 350.

In this regard, the second baffle 390 is open in a direction opposite to the first baffle 380 so that the flow of the water sample from the inlet 340 to the outlet 350 of the measurement chamber 310 is approximately S-shaped.

The first and second vertical baffles 380 and 390, which induce the U-turn flow of the water sample, are placed inside the inlet 340 and outlet 350 of the measurement chamber 310, respectively, and the baffles 380 and 390 are open in opposite directions. Accordingly, the water sample flowing in the measurement chamber has an approximately S-shaped flow, and thus the retention time of the water sample in the measurement chamber can be increased even when the capacity of the measurement chamber is relatively small, and sufficient mixing of the water sample can be achieved, whereby accurate electrical conductivity values without error can be measured in the EC-measuring unit 330.

As described above, in the measurement unit 300 the pH-measuring unit 320 and the EC-measuring unit 330 have improved structures so that they are prevented from coming into contact with air and the water sample. Accordingly, inaccurate date caused by the fluctuation of the measurement unit due to contact with air and a water sample as occurred in the prior art can be avoided and thus more stable and reliable data can be obtained.

Meanwhile, FIG. 7 is a perspective view showing the configuration of a measurement unit 300 not according to the present invention. As in the above cited prior art (Sang-Eun Oh et al.), in the measurement unit 300 of FIG. 7 the pH-measuring unit 320 and the EC-measuring unit 330 are provided inside the reactor body 110.

However, a partitioning plate 150 placed in the reactor body 110 can provide a barrier against the flow of a water sample and air to prevent the water sample and air from impacting the pH-measuring unit 320 and the EC-measuring unit 330 during the flow of the water sample and air.

The partitioning plate 150 is placed horizontally inside the reactor body 110 to divide the reactor body 110 into two spaces (upper region and lower region). The partitioning plate 150 serves as a barrier against the flow of the water sample and air which are directed upward, thereby preventing air bubbles from coming into direct contact with the pH-measuring unit 320 and the EC-measuring unit 330.

The partitioning plate 150 is formed smaller than the horizontal cross-sectional area of the reactor body 110. Thus, an opening 151 through which the partitioning plate 150 and the reactor body 110 are spaced apart from each other is formed. Thus, the opening 151 provides a flow channel through which the water sample can be introduced into the upper region of the reactor body 110, which is separated by the partitioning plate from the lower region in which air aeration occurs.

Thus, due to the partitioning plate 150, the pH-measuring unit 320 and the EC-measuring unit 330 deviate from the path of movement of air bubbles generated by aeration of the water sample and air, which are introduced through the inlet 120 and the air inlet 130, respectively, whereby the air bubbles generated during the flow of the water sample and air can be prevented from coming into contact with and impacting the pH-measuring unit 320 and the EC-measuring unit 330. Accordingly, the pH-measuring unit 320 and the EC-measuring unit 330 can provide more accurate measurement data.

A raw water supply unit 400 contains a water sample and supplies the water sample to the microbial reactor 100 through a first supply pipe 410. In the first supply pipe 410, a first supply pump 411 is provided to supply the water sample to the inlet 120 of the microbial reactor.

A pH-adjusting unit 500 is disposed between the raw water supply unit 400 and the microbial reactor 100 and serves to adjust the pH of the water sample. The pH-adjusting unit 500 monitors the pH of the water sample supplied from the raw water supply unit 400 and adjusts the pH within a reference range. In this regard, the reference range of pH may be in the range of 3-5, which is suitable for survival of sulfur-oxidizing bacteria.

The pH-adjusting unit 500 serves to supply an acidic substance such as sulfuric acid, hydrochloric acid or phosphoric acid to the first supply pipe 410 or to supply an alkaline substance so that the pH of the water sample is adjusted before being introduced into the reactor body 110. Thus, even in the case of wastewater having a very high pH or a very high alkalinity, the detection of toxicity by the microbial reactor is possible.

Meanwhile, although not shown in the figures, the apparatus may further comprise a nutrient element supply unit (not shown) for supplying nutrient elements to the water sample which is supplied to the raw water supply unit 400. The nutrient element supply unit (not shown) is disposed in the first supply pipe 410 and serves to supply carbon sources and micronutrients required for the growth of microorganisms. When the water sample is highly purified water, the nutrient element supply unit supplies small amounts of bicarbonate (NaHCO₃), nitrogen, phosphorus, minerals and the like required for the growth of microorganisms, so that toxicity in the purified water can be detected.

In the case of general stream water, even when the nutrient element supply unit (not shown) does not supply carbon carbons and essential nutrients, accurate experimental results can be obtained, because the stream water contains small amounts of carbon sources and essential nutrients.

In a control unit 600, whether the electrical conductivity (EC) and pH values measured in the measurement unit 300 are below the reference values is determined, and if the measured values are below the reference values, an alarm signal can be issued.

The control unit 600 receives the pH value of the water sample introduced into the microbial reactor 100 and controls the amount of the acidic substance that is supplied from the pH-adjusting unit 500 to the first supply pipe 410.

When stream water has high toxicity, the EC value thereof decreases abruptly, and thus whether the stream water has toxicity can be easily determined using only one reactor without a control for comparison. However, when stream water has a very low toxicity, constant effluent EC appears, and thus it is not easy to determine whether the stream water has low toxicity or no toxicity. For this reason, in this case, comparison with water having no toxicity should be performed.

For this purpose, two or more microbial reactors 100 are installed. Stream water is introduced into the first microbial rector for 0.5-2 hours, and 10-100-fold-diluted stream water as a control is introduced into the second microbial reactor. After 0.5-2 hours, the diluted raw water is introduced into the first microbial reactor which had been introduced with the stream water, and the stream water is introduced into the second microbial reactor. In this way, stream water is introduced alternately into the first reactor and into the second reactor at certain time intervals. Whether the stream water has toxicity can be determined by monitoring the difference between the influent EC and effluent EC of the microbial reactor.

Although it has been shown herein that the apparatus 10 for detecting toxicity in water using microorganisms comprises only one auxiliary microbial reactor 100a, it may comprise two or more auxiliary microbial reactors 100a.

Meanwhile, the apparatus 10 for detecting toxicity in water comprises the auxiliary microbial reactor 100a together with the microbial reactor 100. The auxiliary microbial reactor 100a substitutes for the microbial reactor 100 when the sulfur-oxidizing bacteria present in the microbial reactor 100 was inactivated. Alternatively, the auxiliary microbial reactor 100a serves to supplement sulfur particles coated with sulfur-oxidizing bacteria into the microbial reactor 100, so that the experiment for detecting toxicity in water is continuously performed.

The auxiliary microbial reactor 100a is provided on one side of the microbial reactor 100 and is connected with the second supply pipe 420 of an auxiliary raw water supply unit 400a to receive a water sample. The second supply pipe 420 is connected with the pH-adjusting unit 500 and a nutrient element supply unit (not shown), so that the pH of the water sample can be adjusted or carbon sources and essential nutrients can be supplied to the water sample.

Also, a dilution chamber 700 is connected to the second supply pipe 420 of an auxiliary microbial reactor (not shown). The dilution chamber 700 serves to store synthetic stream water and to supply the synthetic stream water to the water sample supplied through the second supply pipe 420 so that the diluted water sample is supplied to the auxiliary microbial reactor 100a.

The auxiliary microbial reactor 100a is maintained in an environment in which sulfur-oxidizing bacteria can survive, when the microbial reactor 100 is used for detection for toxicity, so that it can substitute for the microbial reactor 100 at any time or can supply active sulfur-oxidizing bacteria to the microbial reactor 100. When the sulfur-oxidizing bacteria present in the microbial reactor 100 were inactivated due to toxic substances, the auxiliary microbial reactor supplies sulfur particles, which have sulfur-oxidizing bacteria attached thereto, to the microbial reactor 100, so that the time taken for sulfur-oxidizing bacteria to adhere to sulfur particles can be reduced and the operation of the microbial reactor 100 can be started again within a short time.

For this purpose, introduction of air into the auxiliary microbial reactor 100a is minimized and the auxiliary microbial reactor 100a receives a diluted water sample obtained by diluting synthetic stream water with a water sample (stream water) at a ratio of 10-100: 1. Also, the hydraulic retention time (HRT) of the sample is increased so that sulfur particles and sulfur-oxidizing bacteria can survive for a long time while reducing the consumption of sulfur.

Hereinafter, the operation and effect of the above-described apparatus 100 for detecting toxicity in water using sulfur-oxidizing bacteria will be described with reference to experimental examples.

For this purpose, actual stream water was introduced into the microbial reactor 100, and the toxicity of the stream water was evaluated based on the change in EC. 50 mL of sulfur particles having a diameter of 2-4 mm was charged into the microbial reactor 100, and air was introduced from the bottom of the reactor through the air inlet 130, and a water sample to be measured was continuously introduced upward into the reactor. The hydraulic retention time (HRT) of the water sample in the reactor was set at 30 minutes, and a shorter retention time is also possible.

To examine the toxicity of the actual stream water, the experiment was carried out by introducing synthetic stream water having no toxicity, and after a certain time, introducing actual stream water. Because the EC value of the actual steam water was somewhat higher than that of the synthetic stream water, small amounts of KCl and MgSO₄ were added to the synthetic stream water so that the synthetic stream water had an EC value substantially identical to that of the actual stream water.

If the pH of the actual stream water is higher than the upper limit of the reference range (3-5), the pH-adjusting unit 500 supplies an acidic or alkaline substance to adjust the pH of the actual stream water.

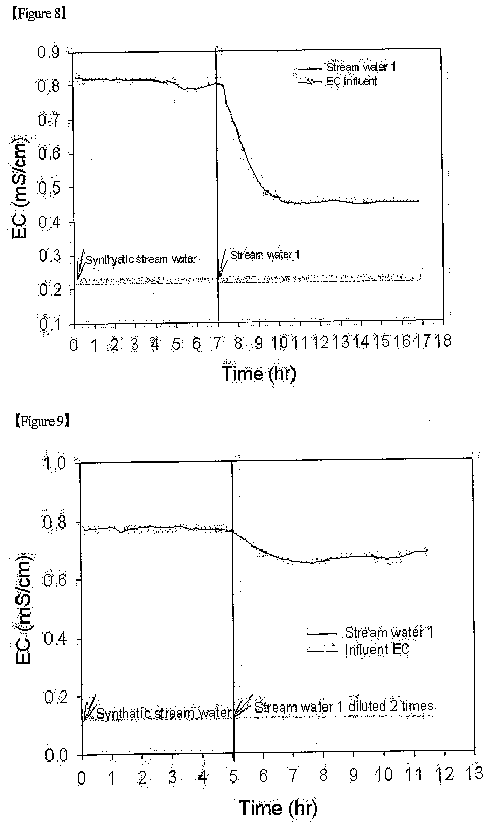

FIG. 8 is a graph showing the change in EC value of effluent water according to the introduction of synthetic stream water and stream water 1 having a high level of contamination (HRT: 30 min; sulfur particles: 2-4 mm; temperature: 30 °C; air flow rate: 150-250 mL/min). Where 10 L of stream water 1 was taken and applied to the toxicity-detecting apparatus 10, synthetic stream water was introduced for 7 hours, and then actual stream water was introduced, and the apparatus was operated at a HRT of 30 min. As a result, the EC value was decreased by 2/3 over 3 hours.

FIG. 9 is a graph showing the change in EC value of effluent water according to the introduction of synthetic stream water and 2-fold-diluted stream water 1 (HRT: 30 min; sulfur particles: 2-4 mm; temperature: 30 °C; air flow rate: 150-250 mL/min). As can be seen therein, when stream water 1 was introduced into the microbial reactor 100, the decrease in the EC value was inhibited.

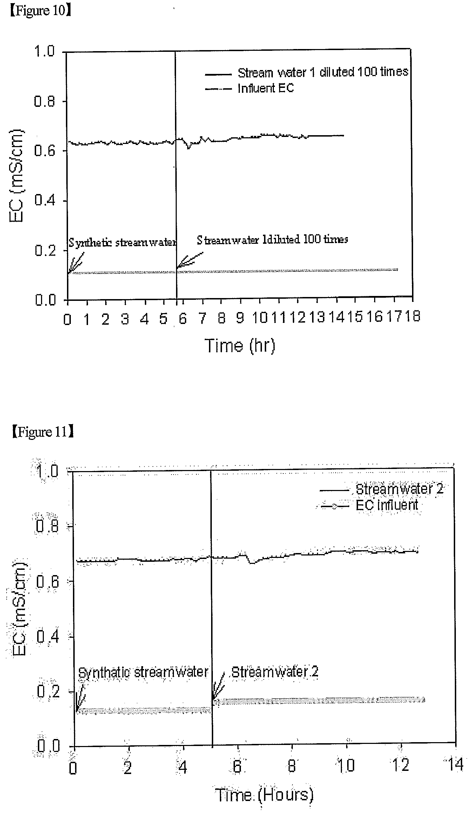

FIG. 10 is a graph showing the change in EC value of effluent water according to the introduction of synthetic stream water and 100-fold-diluted stream water 1 (HRT: 30 min; sulfur particles: 2-4 mm; temperature: 30 °C; air flow rate: 150-250 mL/min). As can be seen therein, the EC of the 100-fold-diluted actual stream water was consistent with the effluent EC of the synthetic stream water. This is because the toxicity of the actual stream water was decreased due to dilution. The actual stream water was analyzed and, as a result, 0.5 mg/L nitrite, 2.6 mg/L n-hexane and 65 ppb dichloromethane were detected as toxic substances.

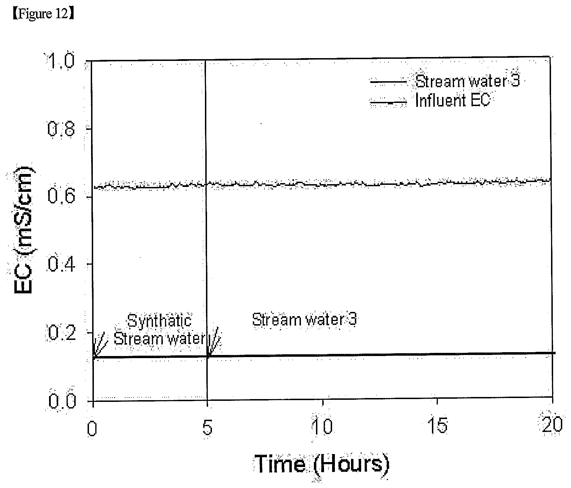

FIG. 11 is a graph showing the change in EC of effluent according to the introduction of synthetic stream water and stream water 2 having no contamination (HRT: 30 min; sulfur particles: 2-4 mm; temperature: 30 °C; air flow rate: 150-250 mL/min), and FIG. 12 is a graph showing the change in EC of effluent water according to the introduction of synthetic stream water and stream water 3 having no contamination (HRT: 30 min; sulfur particles: 2-4 mm; temperature: 30 °C; air flow rate: 150-250 mL/min). As can be seen therein, there was little or no change in the EC of non-toxic stream water 2 and stream water 3. This is believed to be because stream water 2 and stream water 3 contain little or no toxic substances which can affect sulfur-oxidizing bacteria.

Meanwhile, when stream water has high toxicity, the EC value decreases rapidly, and thus whether the stream water has toxicity can be easily determined. However, when stream water has a very low toxicity, determining whether the stream water has toxicity is not easy, and thus comparison with water having no toxicity should be carried out.

For this purpose, two or more microbial reactors 100 are installed, stream water is introduced into microbial reactor 1 for 0.5-2 hours, and 10-100-folded diluted stream water is introduced into microbial reactor 2. After 0.5-2 hours, the diluted stream water is introduced into microbial reactor 1 which had been introduced with the stream water, and the stream water is introduced into microbial reactor 2. In this way, the stream water is introduced alternately into microbial reactor 1 and into microbial reactor 2 at certain time intervals. Also, whether the stream water has toxicity can be determined by monitoring the electrical conductivity (EC) measured in the measurement unit 300.

FIG. 13 is a set of graphs showing the changes in effluent pH and EC upon introduction of 2,000 ppb hexavalent chromium into influent water in the cases of stream water having high toxicity, stream water having low toxicity and stream water having no toxicity (HRT=30 minute; influent pH=7.0 and EC=0.14 mS/cm). In the control unit 600, whether the stream water has toxicity can be determined by monitoring the difference between the influent EC and effluent EC of microbial reactor 1 (R1) and microbial reactor 2 (R2).

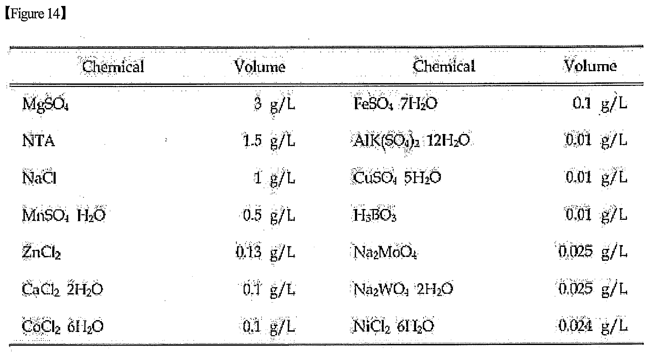

Meanwhile, FIG. 14 is a table showing the composition of minerals used in the preparation of the synthetic stream water.

As described above, the apparatus for detecting toxicity in water using microorganisms comprises the measurement unit in a space separated from the microbial reactor, and thus can prevent the measurement unit from being affected by air bubbles generated during the supply of air and a water sample. Accordingly, more stable and reliable experimental results can be obtained.

Also, where a water sample has high pH or high alkalinity, an acidic or alkaline substance is supplied from the pH-adjusting unit to the water sample to adjust the pH of the water sample within a suitable pH range in which sulfur-oxidizing bacteria can survive, after which the pH-adjusted water sample is supplied to the microbial reactor. Thus, the effect of the pH of the water sample on the activity of sulfur-oxidizing bacteria can be reduced, so that whether the water sample has toxicity can be accurately determined.

In addition, the apparatus further comprises an auxiliary microbial reactor in addition to the microbial reactor, so that, when the activity of sulfur-oxidizing bacteria in the microbial reactor was inhibited, the auxiliary microbial reactor can substitute for the microbial reactor or can immediately supplement sulfur-oxidizing bacteria into the microbial reactor, whereby the experiment for detecting toxicity in water can be continuously performed.

As described above, in the apparatus for detecting toxicity in water using sulfur-oxidizing bacteria the measurement unit for measuring the electrical conductivity and pH of a water sample containing the sulfate ions produced in the microbial reactor is not affected by air bubbles generated when air and the water sample are supplied to the microbial reactor. Thus, more stable and reliable experimental results can be obtained.

Also, in the case where a water sample has high pH or high alkalinity, an acidic or alkaline substance is supplied from the pH-adjusting unit to the water sample to adjust the pH of the water sample within a suitable pH range in which sulfur-oxidizing bacteria can survive, after which the pH-adjusted water sample is supplied to the microbial reactor. Thus, the effect of the pH of the water sample on the activity of sulfur-oxidizing bacteria can be reduced, so that whether the water sample has toxicity can be accurately determined.

In addition, the apparatus further comprises an auxiliary microbial reactor in addition to the microbial reactor, so that, when the activity of sulfur-oxidizing bacteria in the microbial reactor was inhibited, the auxiliary microbial reactor can substitute for the microbial reactor or can immediately supplement sulfur-oxidizing bacteria into the microbial reactor, whereby the experiment for detecting toxicity in water can be continuously performed.

Although the preferred embodiments of the present invention have been described for illustrative purposes, those skilled in the art will appreciate that various modifications, additions and substitutions are possible, without departing from the scope of the invention as disclosed in the accompanying claims.

## Claims

1. An apparatus (10) for detecting toxicity in water using sulfur-oxidizing bacteria, the apparatus comprising:
a microbial reactor (100) comprising a reactor body (110) which contains sulfur-oxidizing bacteria for oxidizing sulfur particles to sulfate ions in the presence of oxygen, an inlet (120) for introducing a water sample into the reactor body (110), and an air inlet (130) for supplying air to the reactor body (110);
a measurement unit (300) for measuring the pH (320) and electrical conductivity (EC) (330) of the water sample containing the sulfate ions produced in the reactor body (110),
wherein the measurement unit (300) is placed in a separated space so as not to be affected by air bubbles generated to supply air to the reactor body (110), the separated space communicating with the reactor body (110) so that the water sample containing the sulfate ions produced in the reactor body (110) is introduced into the measurement unit (300); and
a control unit (600) adapted to compare the electrical conductivity (EC) and pH values measured in the measurement unit (300) with reference values to determine whether the water sample has toxicity,
**characterized in that**
the separated space is defined by a measurement chamber (310) formed integrally with the outer surface of the reactor body (110), and a through hole (311) for passing the water sample is formed in the reactor body (110) provided in the measurement chamber (310).

2. The apparatus of claim 1, further comprising a pH adjusting unit (500) which is placed at one side of the inlet of the microbial reactor (100) and is adapted to supply an acidic substance to the water sample being supplied to the microbial reactor (100) so as to adjust the pH of the water sample within a reference range.

3. The apparatus of claim 2, further comprising a nutrient element supply unit which is placed on one side of the inlet of the microbial reactor (100) and is adapted to supply a nutrient element to the water sample being supplied to the microbial reactor (100) so as to maintain the activity of the sulfur-oxidizing bacteria.

4. The apparatus of claim 2, further comprising an auxiliary microbial reactor (100) which is placed on one side of the microbial reactor (100) and contains active sulfur-oxidizing bacteria, in which, when the sulfur-oxidizing bacteria in the microbial reactor (100) were inactivated, the auxiliary microbial reactor (100) is adapted either to detect toxicity in the water sample supplied from the microbial reactor (100) or to supplement oxidizing bacteria-coated sulfur particles thereof into the microbial reactor (100), thereby determining whether the water sample has toxicity.

5. The apparatus of claim 4, wherein the auxiliary microbial reactor (100) is adapted to supplying minimum amounts of water and air thereto while the microbial reactor (100) is adapted to detect toxicity in the water sample.

6. The apparatus of claim 2, further comprising an auxiliary microbial reactor (100) which is placed on one side of the microbial reactor (100), in which the auxiliary microbial reactor (100) and the microbial reactor (100) are adapted to be alternately supplied with the water sample and a diluted water sample to determine whether the water sample has toxicity.

## Patentansprüche

1. Vorrichtung (10) zum Feststellen von Toxizität in Wasser unter Anwendung von schwefeloxidierten Bakterien, wobei die Vorrichtung Folgendes umfasst:
einen mikrobiellen Reaktor (100), der einen Reaktorkörper (110) umfasst, der schwefeloxidierte Bakterien zum Oxidieren von Schwefelteilchen zu Sulfationen in Gegenwart von Sauerstoff enthält, einen Einlass (120) zum Einführen einer Wasserprobe in den Reaktorkörper (110) und einen Lufteinlass (130) zum Liefern von Luft an den Reaktorkörper (110);
eine Messeinheit (300) zum Messen des pH-Werts (320) und der elektrischen Leitfähigkeit (EC) (330) der Wasserprobe, die die in dem Reaktorkörper (110) hergestellten Sulfationen enthält,
wobei die Messeinheit (300) in einen separaten Raum positioniert ist, um nicht durch Luftblasen, die zum Liefern von Luft zum Reaktionskörper (110) erzeugt werden, beeinflusst zu werden, wobei der separate Raum mit dem Reaktorkörper (110) kommuniziert, so dass die Wasserprobe, die die in dem Reaktorkörper (110) hergestellten Sulfationen enthält, in die Messeinheit (300) eingeführt wird; und
eine Reguliereinheit (600), die geeignet ist, die elektrische Leitfähigkeit (EC) und die pH-Werte, die in der Messeinheit (300) gemessen werden, mit Bezugswerten zu vergleichen, um zu bestimmen, ob die Wasserprobe Toxizität aufweist,
**dadurch gekennzeichnet, dass**
der separate Raum durch eine Messkammer (310) definiert ist, die integral mit der Außenfläche des Reaktorkörpers (110) gebildet ist und ein durchgehendes Loch (311) zum Hindurchführen der Wasserprobe in dem Reaktorkörper (110) gebildet ist, der in der Messkammer (310) bereitgestellt ist.

2. Vorrichtung nach Anspruch 1, ferner eine pH-Wert-Einstellungseinheit (500) umfassend, die auf eine Seite des Einlasse des mikrobiellen Reaktors (100) positioniert und geeignet ist, eine saure Substanz zu der Wasserprobe, die zu dem mikrobiellen Reaktor (100) geliefert wird, zu liefern, um den pH-Wert der Wasserprobe innerhalb eines Bezugsbereichs einzustellen.

3. Vorrichtung nach Anspruch 2, ferner eine Nährstoffelementliefereinheit umfassend, die auf eine Seite des Einlasse des mikrobiellen Reaktors (100) positioniert und geeignet ist, ein Nährstoffelement zu der Wasserprobe, die zu dem mikrobiellen Reaktor (100) geliefert wird, zu liefern, um die Aktivität der schwefeloxidierten Bakterien aufrechtzuerhalten.

4. Vorrichtung nach Anspruch 2, ferner einen mikrobiellen Hilfsreaktor (100) umfassend, der auf eine Seite des mikrobiellen Reaktors (100) positioniert ist und aktive schwefeloxidierte Bakterien enthält,
wobei, wenn die schwefeloxidierten Bakterien in dem mikrobiellen Reaktor (100) aktiviert worden sind, der mikrobielle Hilfsreaktor (100) geeignet ist, entweder die Toxizität in der Wasserprobe, die zu dem mikrobiellen Reaktor (100) geliefert wird, festzustellen oder mit oxidierenden Bakterien beschichtete Schwefelteilchen davon in dem mikrobiellen Reaktor (100) aufzufüllen, um dadurch zu bestimmen, ob die Wasserprobe Toxizität aufweist.

5. Vorrichtung nach Anspruch 4, wobei der mikrobielle Hilfsreaktor (100) geeignet ist, Mindestmengen von Wasser und Luft dazu zu liefern, während der mikrobielle Reaktor (100) geeignet ist, Toxizität in der Wasserprobe festzustellen.

6. Vorrichtung nach Anspruch 2, ferner einen mikrobiellen Hilfsreaktor (100) umfassend, der auf eine Seite des mikrobiellen Reaktors (100) positioniert ist, wobei der mikrobielle Hilfsreaktor (100) und der mikrobielle Reaktor (100) geeignet sind, abwechselnd mit der Wasserprobe und einer verdünnten Wasserprobe beliefert zu werden, um zu bestimmen, ob die Wasserprobe Toxizität aufweist.

## Revendications

1. Appareil (10) pour détecter une toxicité dans de l'eau utilisant des bactéries oxydatrices de soufre, l'appareil comprenant :
un réacteur microbien (100) comprenant un corps de réacteur (110) qui contient des bactéries oxydatrices de soufre pour oxyder des particules de soufre en ions sulfate en présence d'oxygène, une entrée (120) pour introduire un échantillon d'eau dans le corps de réacteur (110) et une entrée d'air (130) pour approvisionner en air le corps de réacteur (110) ;
une unité de mesure (300) pour mesurer le pH (320) et la conductivité électrique (EC) (330) de l'échantillon d'eau contenant les ions sulfate produits dans le corps de réacteur (110),
où l'unité de mesure (300) est placée dans un espace séparé de manière à ne pas être affectée par les bulles d'air produites pour approvisionner l'air au corps de réacteur (110), l'espace séparé communiquant avec le corps de réacteur (110) de sorte que l'échantillon d'eau contenant les ions sulfate produits dans le corps de réacteur (110) est introduit dans l'unité de mesure (300) ; et
une unité de contrôle (600) adaptée pour comparer les valeurs de conductivité électrique (EC) et de pH mesurées dans l'unité de mesure (300) avec des valeurs de référence pour déterminer si l'échantillon d'eau présente une toxicité,
**caractérisé en ce que**
l'espace séparé est défini par une chambre de mesure (310) formée intégralement avec la surface externe du corps de réacteur (110), et un orifice (311) pour faire passer l'échantillon d'eau est formé dans le corps de réacteur (110) disposé dans la chambre de mesure (310).

2. Appareil selon la revendication 1, comprenant en outre une unité d'ajustement de pH (500) qui est placée à un côté de l'entrée du réacteur microbien (100) et est adaptée pour approvisionner une substance acide à l'échantillon d'eau qui est approvisionné au réacteur microbien (100) de manière à ajuster le pH de l'échantillon d'eau dans une plage de référence.

3. Appareil selon la revendication 2, comprenant en outre une unité d'approvisionnement d'élément nutritif qui est placée sur un côté de l'entrée du réacteur microbien (100) et est adaptée pour approvisionner un élément nutritif à l'échantillon d'eau qui est approvisionné au réacteur microbien (100) de manière à maintenir l'activité des bactéries oxydatrices de soufre.

4. Appareil selon la revendication 2, comprenant en outre un réacteur microbien auxiliaire (100) qui est placé sur un côté du réacteur microbien (100) et contient des bactéries oxydatrices de soufre actives,
dans lequel, quand les bactéries oxydatrices de soufre dans le réacteur microbien (100) ont été inactivées, le réacteur microbien auxiliaire (100) est adapté pour détecter la toxicité dans l'échantillon d'eau approvisionné du réacteur microbien (100) ou pour complémenter leurs particules de soufre revêtues de bactéries oxydatrices dans le réacteur microbien (100), déterminant ainsi si l'échantillon d'eau présente une toxicité.

5. Appareil selon la revendication 4, dans lequel le réacteur microbien auxiliaire (100) est adapté pour approvisionner des quantités minimum d'eau et d'air à celui-ci alors que le réacteur microbien (100) est adapté pour détecter une toxicité dans l'échantillon d'eau.

6. Appareil selon la revendication 2, comprenant en outre un réacteur microbien auxiliaire (100) qui est placé sur un côté du réacteur microbien (100), dans lequel le réacteur microbien auxiliaire (100) et le réacteur microbien (100) sont adaptés pour être approvisionnés en alternance avec l'échantillon d'eau et un échantillon d'eau diluée pour déterminer si l'échantillon d'eau présente une toxicité.
